# EUROPEAN PATENT SPECIFICATION

(11) **EP 1 942 976 B1**
(45) Date of publication and mention of the grant of the patent: **29.06.2011**
(21) Application number: 06809095.0
(22) Date of filing: 13.10.2006
(51) Int. Cl.: A61M 29/00

(54) **BALLOON CATHETER SYSTEM FOR TREATING VASCULAR OCCLUSIONS**
BALLONKATHETERSYSTEM ZUR BEHANDLUNG VON GEFÄSSVERSCHLÜSSEN
SYSTEME DE CATHETER A BALLONNET SERVANT A TRAITER LES OCCLUSIONS VASCULAIRES

(30) Priority: 14.10.2005 US 726180 P
(43) Date of publication of application: 16.07.2008
(60) Divisional application: 10196778.4
(73) Proprietor: ENDOCROSS, 20692 Yoknean (IL)
(72) Inventor: HIRSZOWICZ, Eran, 52236 Ramat-gan (IL); LEVIT, Eran, 25147 Kfar Vradim (IL); DUBI, Shay, 69513 Tel-aviv (IL)
(74) Representative: Turner, Craig Robert
(86) International application number: PCT/IB2006/002958
(87) International publication number: WO 2007/042936

(56) References cited:
- EP-A- 0 987 045
- US-A- 4 820 270
- US-A- 5 514 093
- US-A- 5 554 120
- US-A1- 2004 133 263

## Description

### Field of the invention

The present invention relates to a balloon catheter system. More specifically, the present invention is a crossing balloon system (CBS) for use in the treatment of chronic total occlusion (CTO) and other occlusive conditions of blood vessels.

### Background of the invention

Chronic total occlusion of a blood vessel is, as the name suggests, a condition in which there is complete (or near complete) obstruction of that vessel due to the development of an intravascular lesion comprising atheromatous plaque material and/or thrombotic material. Between 10 and 20 percent of patients undergoing percutaneous coronary interventions (PCI) have CTO. Successful opening of CTO lesions improves anginal status, increases exercise capacity, and reduces the need for bypass surgery. However, PCI of cases of CTO have historically posed problems, with lower success rates (40 to 80 percent - average 60 percent), higher equipment costs, and a higher restenosis rate. When MACE (Major Arterial or Cardiac Events) is taken into account, the success rate typically in the range of 20 to 30 percent.

Conventional intervention tools such as angioplasty balloons are often too flexible or blunt to cross the CTO site, which often contains extremely hard, calcified tissue that may form an impenetrable barrier to the advancement of a guidewire therethrough. Even a less than total occlusion may contain complex structures which may trap or divert the steering end of the guidewire. In view of the great difficulties encountered in attempting to properly position a guidewire across the stenosis, conventional guided atherectomy or dilatation devices such as cutting elements and balloons cannot be used to cross the lesion as long as a guidewire was not inserted through the lesion since they rely on complete wire crossability .

A further problem associated with the use of conventional devices is the risk of perforating the blood vessel being treated. For example, a guidewire or cutting tool, when advanced, may cause dissection of the tissues of the arterial wall instead of the occlusion, thereby creating a false lumen and possibly perforating the artery. If enough blood from a perforated artery accumulates in the pericardial space surrounding the heart, it will result in a condition known as cardiac tamponade in which the heart is compressed and emergency surgical intervention is required to avert heart failure and death.

Another reason that conventional types of apparatus are typically ineffective in treating total or near total occlusions is that conventional balloon catheter shafts and guidewires do not perform well under the compressive loading and torque loading that are required in order to advance such devices across a CTO lesion.

Statistically, the predominant reason for failure to open CTO lesions with PCI has been failure to cross the lesion with a guidewire (80 percent) and failure of a balloon to track along the guidewire (15 percent) through the very hard lesion. Many types of guidewires and devices have been tried, but successful recanalization has remained at about 60 percent. Crossing CTO lesions in patients with peripheral vascular disease has met with similar problems, for example, the reported success rate for percutaneous catheter-based treatment of chronic subclavian artery occlusion being in the range of 46%-83%

US 2004/0133263 relates to an assembly for delivery and deployment of an inflation extendable stent within a vessel comprising inner and outer conduits with an inflatable balloon connected therebetween. EP 0987045 relates to a balloon dilatation catheter that may be used for percutaneous transluminal coronary angioplasty and includes a coaxial construction of an inner tube and an outer tube.

It is therefore an object of the present invention to provide a balloon catheter system that is capable of penetrating and crossing CTO lesions.

Another object of the present invention is to provide a CTO-crossing system that will minimize trauma to the vascular wall.

A further object of the present, invention is to provide a balloon catheter system having the aforementioned advantages together with the additional advantage of being relatively easy to operate in the hands of healthcare professionals with experience with conventional atherectomy and dilation systems.

Further objects and advantages of the present invention- will become apparent as the description proceeds.

### Summary of the Invention

It has now been found by the inventors that it is possible to achieve the above-mentioned objects by means of the use of a balloon catheter system fitted with a balloon that is terminally infolded (or a balloon which may be caused to adopt such an intussuscepted configuration). Following delivery of the balloon to the proximal side of the occluding vascular lesion to be treated, said balloon is inflated such that it becomes anchored within the blood vessel. The balloon is then caused to rapidly inflate and partially deflate in a cyclic manner, such that said balloon alternately elongates and axially contracts. The distal portion of the catheter shaft upon which the balloon is mounted, and which projects beyond the distal margin of said balloon is thereby caused to similarly oscillate along a distal-proximal axis (wherein proximal is defined as the direction towards the operator and distal as the direction away from the operator). This longitudinal oscillation of the catheter shaft in proximity to the occluding lesion to be treated causes damage and break-up of the lesion, ultimately enabling the operator to advance the balloon catheter and/or other conventional angioplasty devices across the lesion.

During the course of their work, the inventors further found that in order to facilitate oscillation of the catheter shaft along a distal-proximal axis in response to the similar elongation-contraction of the balloon, it is desirable that said catheter shaft contains at least one portion that is elastically deformable along said distal-proximal axis. Without wishing to be bound by theory, it is believed that in response to increased pressure inside the proximally intussuscepted balloon, the folded distal balloon tapers are extended in a distal direction thereby exerting tension forces along the catheter shaft. Due to the elasticity of the shaft, the induced tension forces result in elongation of the said shaft, thereby stressing the shaft, similar to a tension spring charging procedure. Partial pressure release inside the balloon reduces these tension forces. The catheter shaft then acts as a returning spring and thereby assists in rolling the distal balloon tapers back to their initial position.

The present invention is therefore primarily directed to a balloon catheter comprising:
a hollow inner shaft disposed within a hollow outer shaft such that the distal end of the inner shaft extends beyond the distal end of the outer shaft, wherein the lumen of said inner shaft is suitable for allowing the passage of a guidewire through all or part of its length;
a balloon attached at its proximal end to said outer shaft and at its distal end to said inner shaft; and
means for the introduction of an inflation fluid into the annular space formed between the inner surface of the outer shaft and the outer surface of the inner shaft and therefrom into the lumen of said balloon, and for the removal thereof;
characterised in that the inner shaft is constructed such that is comprises at least one portion that is elastically deformable such that following radial expansion of the balloon to a first expanded state, said inner shaft is capable of responding to further longitudinal expansion of the balloon to a second expanded state by increasing its length from a resting value, and of responding to subsequent partial deflation back to said first expanded state by reducing its length back to said resting value.

The inner shaft of the balloon catheter defined hereinabove comprises at least one portion that is elastically deformable.

In one preferred embodiment of the balloon catheter described hereinabove, following radial expansion of the balloon to a first expanded state, said elastically deformable portion of the inner shaft is capable of responding to further longitudinal expansion of the balloon to a second expanded state by increasing its length from a resting value, and of responding to subsequent partial deflation back to said first expanded state by reducing its length back to said resting value.

According to one preferred embodiment of the balloon catheter of the invention, the distance between the points of attachment of the balloon to the inner and outer shafts is less than the overall length of the balloon.

According to another preferred embodiment of the balloon catheter defined hereinabove, the balloon is attached at its distal end in an inverted manner, such that the external surface of said balloon is in contact with, and attached to, the outer surface of the inner shaft.

In yet another preferred embodiment, the balloon is attached at its distal end in a non-inverted manner, such that the internal surface of said balloon is in contact with, and attached to, the outer surface of the inner shaft.

In one particularly preferred embodiment, the aforementioned at least one elastically deformable portion is constructed of a blend of nylon and Pebax.

The balloon catheter defined hereinabove may be constructed either in the form of an over-the-wire catheter or as a rapid exchange (single operator) catheter. Both of these embodiments will be described in detail hereinbelow.

In another preferred embodiment of the invention, the balloon catheter disclosed hereinabove, the inner shaft is capable of being moved along its longitudinal axis in relation to the outer shaft, and wherein said catheter further comprises means for immobilizing said inner shaft.

A balloon catheter comprises a conduit having a proximal portion and a distal portion,
wherein said proximal portion contains two separate lumens, one of said lumens being suitable for allowing the passage of a guidewire therethrough, the other lumen being suitable for allowing passage of a fluid,
wherein said distal portion is a single-lumen conduit that is in fluid communication with the guidewire lumen of said proximal portion,
wherein a balloon is attached at its proximal end to the outer surface of said proximal portion and at its distal end to said distal portion,
and wherein at least part of said distal portion is constructed such that following radial expansion of the balloon to a first expanded state, said distal conduit is capable of responding to further expansion of the balloon to a second expanded state by increasing its length from a resting value, and of responding to subsequent partial deflation back to said first expanded state by reducing its length back to said resting value.

In a particularly preferred embodiment of the device disclosed immediately hereinabove, the distal portion comprises at least one portion that is elastically deformable. In a particularly preferred embodiment of this implementation, the at least one elastically deformable portion is constructed of a blend of nylon and Pebax.

There are following modifications of the embodiment described immediately hereinabove, in which the length of the flexible distal portion need not be limited to the length of the balloon as is the case in said embodiment.

In the first of these modifications, the proximal balloon neck is longer than in the previously-described embodiment (and indeed is substantially longer than the proximal balloon neck of conventional angioplastic balloons) thereby permitting the use of a longer distal portion within the abovementioned conduit construction.

In the second of these modifications, the catheter further comprises a connecting tube segment positioned between the conduit proximal portion and the proximal balloon neck, thereby forming a middle portion, wherein said middle portion comprises two concentrically arranged conduits, the lumen of the outer of said concentrically arranged conduits being in fluid communication with the fluid passage lumen of said proximal portion, the inner of said concentrically arranged conduits being formed by the distal conduit, wherein the lumen of said distal conduit is in fluid communication with the guidewire lumen of said proximal portion, the connecting tube segment having a proximal end attached to the distal end of the proximal portion, and a distal end attached to the balloon proximal neck.

In particularly preferred embodiments of both of the modifications of the previously-described implementation, the distal portion comprises at least one portion that is elastically deformable. In a particularly preferred embodiment of this implementation, the at least one elastically deformable portion is constructed of a blend of nylon and Pebax.

A method for treating vascular occlusions in a patient in need of such treatment, comprises the steps of:
a) bringing a balloon into proximity with a vascular occlusion within a blood vessel to be treated;
b) causing said balloon to be cyclically inflated and partially deflated such that an element attached to said balloon is caused to oscillate along a distal-proximal axis in close proximity to the vascular occlusion, thereby causing complete or partial fracture of said occlusion.

In a particularly preferred embodiment of this method, the element attached to the balloon is a portion of a catheter shaft. Preferably, this portion is the distal most portion of the catheter (i.e. the inner shaft of the first embodiment described hereinabove, or the distal portion of the conduit described in the immediately preceding two embodiments).

Preferably, prior to the cyclical inflation and partial deflation of the balloon, said balloon is inflated to a first pressure, such that said balloon becomes anchored within the blood vessel being treated, and such that during the step of cyclical inflation and partial deflation, the balloon is further inflated by increasing its internal pressure from said first pressure to a second pressure, and partially deflated by reducing its internal pressure from said second pressure to said first pressure.

In one implementation of the method described hereinbefore, subsequent to anchoring of the balloon within the blood vessel, said method further comprises the step of moving the inner catheter shaft in a proximal direction such that the distal extremity of the balloon becomes intussuscepted. This particular embodiment of the method is generally used in conjunction with balloons that are manufactured such that they do not inherently possess an intussuscepted portion ("non- charged" balloons, as described in more detail hereinafter) . The proximal movement of the inner shaft, as mentioned above, thereby causes the formation of an intussusception in the previously non-intussuscepted balloon.

### Brief Description of the Drawings

The present invention is illustrated by way of example in the accompanying drawings, in which similar references consistently indicate similar elements and in which:
- Fig. 1 schematically illustrates an over the wire implementation of the balloon catheter of the invention;
- Fig. 2 schematically illustrates a rapid exchange implementation of the balloon catheter of the invention;
- Fig. 3 demonstrates various elastic shaft implementations according to preferred embodiments of the invention;
- Fig 4 schematically illustrates various distal tip implementations that may be used in the balloon catheter of the invention;
- Figs. 5A and 5B schematically illustrates alternative balloon configurations that may be used in the balloon catheter of the invention;
- Fig. 6 schematically illustrates an implementation of the balloon catheter of the invention wherein an auxiliary tube is used instead of the inner guide wire tube;
- Figs. 7A to 7F demonstrate one possible procedure for opening a path through an occluded vessel;
- Figs. 8A and 8B demonstrate another possible procedure for opening a path through an occluded vessel;
- Figs. 9A to 9C illustrate three different embodiments of the balloon catheter of the present invention using a bi- lumen conduit instead of a concentric inner tube-outer tube configuration proximal to the balloon attachment; and
- Figs. 10A to 10E illustrate a method of producing a balloon catheter of the present invention having an intussuscepted distal balloon attachment.

### Detailed Description of Preferred Embodiments

The present invention provides a device for the treatment of vascular occlusions by means of disrupting vascular occlusions (particularly in cases of CTO) or other blockages formed within blood vessels in order to provide pathways for the placement of interventional devices and catheters as part of an overall effort to restore normal circulatory function. In general terms, the catheter device of the present invention achieves its objectives by creating a path with the least possible mechanical resistance through or around the occlusion. Thus, the presently disclosed device includes a distally-advanceable inner shaft tip which is caused to rapidly move back and forth (i.e. distally and proximally), thereby "ramming" the lesion. In addition, the device comprises an inflatable balloon for anchoring the catheter inside the vessel.

In one preferred mode of operation, the device of the present invention creates the aforementioned path of least resistance by means of mechanically fracturing the vascular occlusion, while at the same time, greatly minimizing the risk of perforating the endothelia of the vascular wall. The latter advantage is achieved, in part, by virtue of the fact that the distal tip of the inner catheter shaft (as will be described in more detail hereinbelow) actually moves a very short distance (distally and proximally), thereby reducing the possibility that said tip will deviate from its centered position and motion.

In another aspect of the invention, following disruption of the occluding lesion, the pathway thereby created through said lesion is used to accommodate the conventional angioplasty balloon feature of the catheter, in order to simultaneously treat the vessel using conventional balloon angioplasty methods as part of an overall effort to restore normal circulatory function within the blood vessel.

In its most general form, the crossing balloon system disclosed and described herein comprises a novel balloon catheter, the fluid pressure inside of which may be rapidly increased and decreased by means of a pressure generator console connected thereto.

The balloon catheter of the present invention comprises a flexible inner catheter shaft fitted within a rigid outer shaft. The distal portion of the catheter defines an inflation lumen, as will be described in more detail hereinbelow. A balloon is connected at its proximal end to the distal end of the outer shaft section and at its distal end to the inner shaft, and is in fluid communication with the inflation lumen.

The manner in which the distal tapered extremity of the balloon is affixed to the distal end of the flexible inner catheter shaft permits the distal end of said balloon to roll and expand in response to increased pressure inside the catheter system. Similarly, as a result of this pressure increase, the inner shaft is caused to be stretched distally. Subsequently, when the pressure inside the catheter system is reduced, the elasticity of the inner shaft causes retraction (i.e. in a proximal direction) of the inner -shaft tip to its original position in response to decreased pressure. A rapid, reciprocating pressure cycle (having a frequency in the sonic or subsonic range) thus causes a correspondingly rapid linear oscillatory motion of the distal tip of the inner catheter shaft. In this way, the rapid cyclical distal-proximal movement of the inner shaft tip, together with the shock waves set up within the volume of blood situated between the inner shaft tip and the obstruction, may be used to progressively cut through an intravascular lesion located in the region of the inner shaft tip.

As mentioned hereinabove, the ability of the distal end of the balloon to roll and expand in response to increased pressure inside the catheter system is determined by the manner in which said distal end is affixed to the inner shaft. Essentially, the distal end of the balloon needs to be attached to the inner shaft in such a way that, during the part of the method of use wherein said balloon is caused to oscillate, said distal end is intussuscepted. This may be achieved in two different ways:

### I. Pre-charged balloon conformation

In this conformation, the balloon is attached to the distal end of the inner shaft during manufacture such that its distal end is always intussuscepted (i.e. inwardly-folded onto the catheter shaft. This conformation may be achieved in a number of different ways as will be discussed further hereinbelow.

### II. Non-charged balloon conformation

In this conformation, the distal end of the balloon is attached to the inner shaft of the catheter in a conventional, non-intussuscepted manner. The distal intussusception is then created by the operator by means of moving the inner shaft in a proximal direction (in relation to the outer shaft). The inner shaft is then locked in place, thereby preserving the distal intussusception created by this procedure.

The balloon catheter of the present invention may be constructed as an over-the-wire catheter or as a single-operator (i.e. rapid exchange) catheter. In addition, the catheter may also be manufactured using bi-lumen catheter tubing (for at least a portion of the total length of the catheter), as will be described hereinbelow.

In a preferred embodiment, the aforementioned balloon catheter is manufactured as a sterile, single use catheter, which is entirely disposable.

The balloon catheter is, as mentioned hereinabove, connected to a reusable pressure generator console, wherein said console comprises a pressure pump, a pressure adjustment interface and a display providing control information for the physician. In one embodiment, the pressure generator console includes a piston and a chamber with an actuation member attached to the piston. The chamber may be used to introduce inflation fluid (e.g. contrast material and saline solution) into the pressure generator and the inflation lumen. A pressure sensor/gauge and a balloon sizing scale may be incorporated into the catheter assembly to assist the treating physician in monitoring the procedure. A longitudinally oscillating drive, such as a solenoid and/or a rotary electrical motor, may be operatively connected to the pressure generator.

The procedure begins by advancing a guidewire within a blood vessel to a vascular occlusion. The catheter is advanced over the guidewire so that the distal end of the catheter is in proximity to the vascular occlusion. The guidewire is slightly retracted from the catheter tip. The balloon, the distal tip of which is located just proximal to the lesion to be treated, is then inflated to a first inflation pressure (anchoring pressure) which causes the balloon to be anchored within the confines of the blood vessel. Preferably, a symmetrical balloon inflation shape is used in order to ensure that the tip of the catheter is centered within the vessel in front of the occlusion. In non-charged versions of the device, the physician can manipulate the inflated balloon by releasing a grasping element that allows the inner shaft to be moved relative to outer shaft. The inner shaft is then retracted proximally and anchored at its new location by re-applying the grasping element. Proximal retraction of inner shaft folds the distal end of balloon inwardly and shortens the balloon's length (i.e. causes intussusception). If required, the operator may then advance the balloon catheter device distally in order to diminish the distance between distal tip and the occlusion. This is preferably carried out by partially deflating the balloon, thereby releasing its anchor in vessel, and advancing the device distally until the catheter's tip contacts the occlusion.

After re-anchoring the balloon in its new position at the treatment site, the user may operate the device in a vibrating mode by applying an oscillating pressure source to open a passage through the occlusion. During the phase of the oscillatory cycle wherein the balloon pressure is increased from the anchoring pressure to a higher pressure, the elastic inner shaft extends and allows the distal balloon taper to roll and advance the catheter inner shaft tip in a forwards (i.e. distal) direction. Subsequently, during the phase of the oscillator cycle wherein the balloon pressure is reduced back to the anchoring pressure, the elastic properties of the inner shaft will cause said shaft to move in a reverse (i.e. proximal) direction. This rapid, cyclical increase and decrease in fluid pressure that is produced by the pressure generator console thus results in a rapid distal-proximal linear motion of the inner shaft tip. This motion takes place over only a very short distance in order to keep the inner shaft tip centered within the vessel lumen. After the lesion in front of the catheter has been treated (i.e. rammed, scored and/or fractured), the balloon is deflated, advanced further distally through the lesion and the procedure is then repeated, thereby traumatizing the next portion of the lesion. Once the operator has succeeded in crossing the lesion with the guidewire the catheter system may then be further used to dilate the lesion and create a passage for a stent or a larger diameter balloon dilatation catheter, using conventional angioplastic techniques that are well known in the art.

The method of the present invention may be used as the primary or sole means for crossing a CTO lesion. Alternatively, it may be employed after an unsuccessful attempt to cross the lesion using a conventional guidewire or cutting tool method.

Several preferred embodiments of the device of the present invention will now be described in more detail, with reference to the accompanying drawings. It will, of course, be understood that the particular embodiments described herein are brought for the purpose of illustration only, and that the scope of the present invention is not limited to these specific embodiments alone.

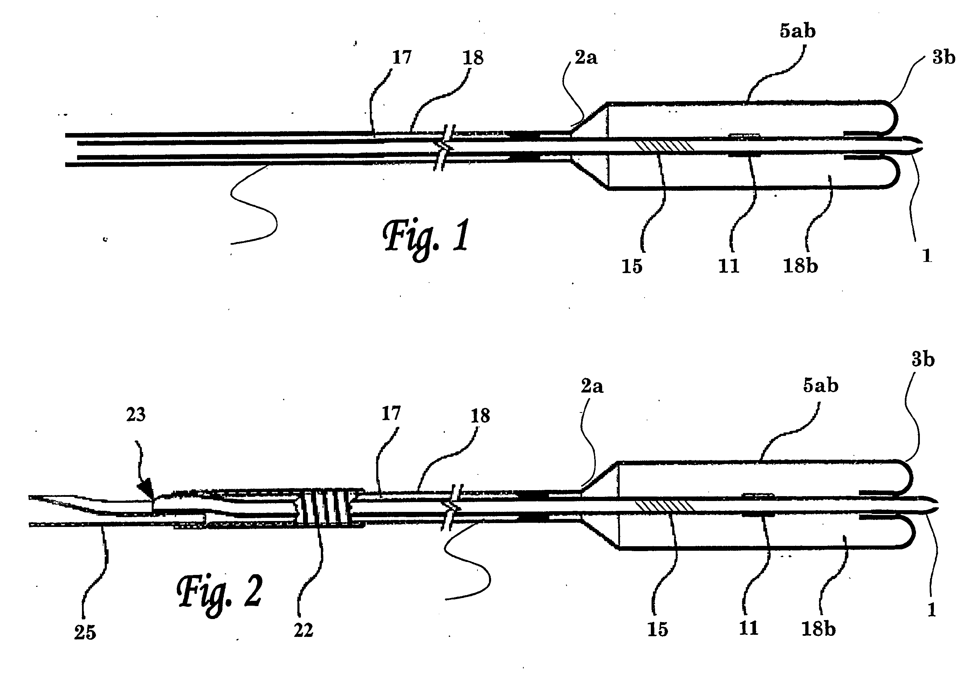

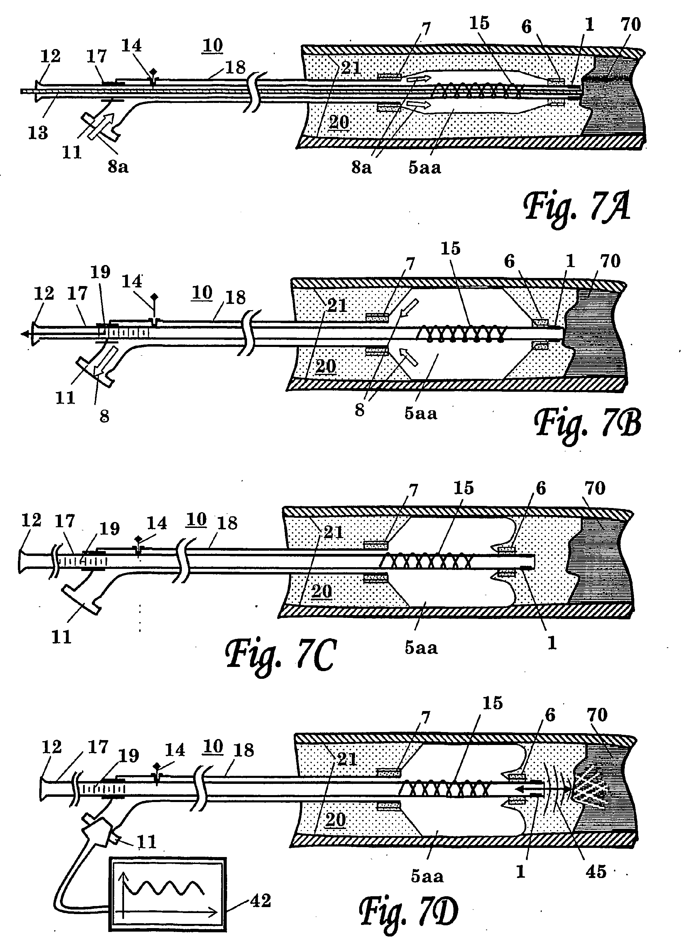

Fig. 1 schematically illustrates an over the wire implementation of the balloon catheter of the invention. This balloon catheter implementation comprises an outer shaft **18,** inner shaft **17** passing thereinside, and a balloon 5ab. The lumen of inner shaft 17 may be used for passing a guide wire thereinside, which may be introduced via its proximal opening (e.g., **12** in Figs. 7A-7F). In the pre-charged embodiment shown in this figure, balloon **5ab** has a conical proximal end **2a** which tapers proximally towards its annular attachment area on the outer surface of the distal end portion of outer shaft **18,** and a rounded distal end **3b** which is obtained by folding the distal end of balloon **5ab** proximally inwardly and by attaching the outer surface of its distal end portion to an annular attachment area on the outer surface of the distal end portion of inner shaft **17.** Other types of balloon attachment (resulting in either pre-charged or non-charged balloons) are also possible, one example of which is described in more detail hereinbelow.

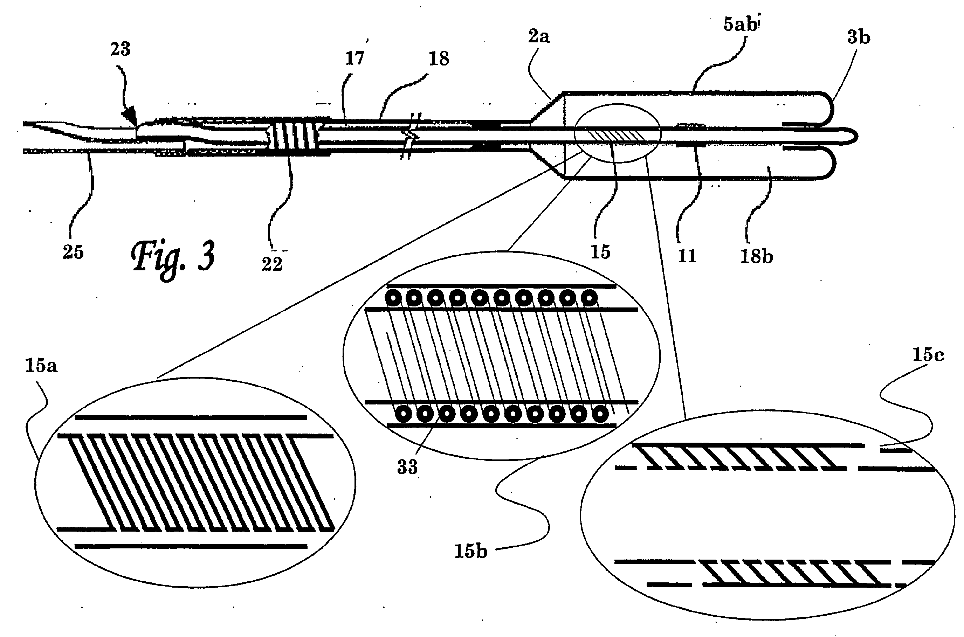

Inner shaft **17** is manufactured either from an elastic material or from an essentially non-elastic material that incorporates at least one elastic portion **15** along its length. Of course elastic portions **15** may be obtained in many various ways, as will be exemplified hereinafter with reference to Figs. 3. Inner shaft **17** may further comprise a radiopaque marker **11.** The distal tip **1** of inner shaft **17** is preferably made rigid to allow using it for opening a passage via an occluded vessel. Inflation fluid lumen **18a** (as indicated in Fig. 2) obtained between the inner shaft **17** and the inner wall of outer shaft 18 provides a path for filling the inner space **18b** of balloon **5ab** with pressurized inflation fluid provided therethrough.

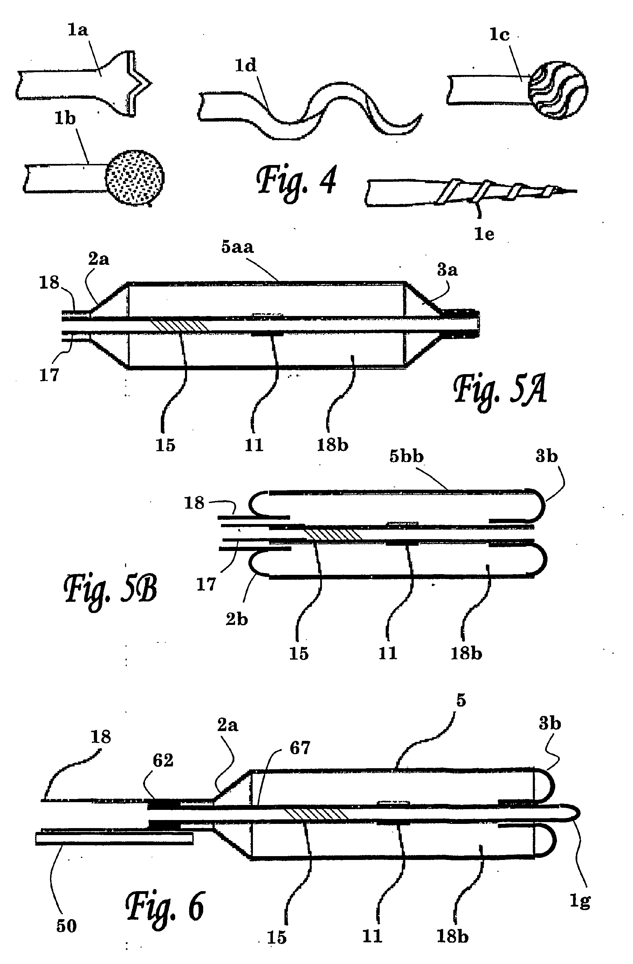

In a typical procedure the balloon catheter is inserted and advanced through the patient's vessels in a deflated state towards a treatment site which may comprise an occlusion. After reaching the treatment site inflation fluids are pressurized via inflation fluid lumen **18a** and fill inner space **18b** of balloon **5ab.** The wall of the inflated balloon is pressed against the inner wall of the blood vessel, thereby anchoring it at the treatment site. In the case of a catheter utilizing a non-charged balloon (e.g. the balloon depicted in Fig. 5A), in order to operate said catheter in its vibrating mode the inner shaft **17** is slightly retracted proximally (e.g., about 3 mm) and affixed in its displaced location. Proximal retraction of inner shaft **17** cause distal end portion **3b** of balloon **5ab** to collapse proximally inwardly on the outer surface of distal end portion of inner shaft **17,** thereby shortening the balloon's length and reducing its volume. Portions of the inflation fluid may be discharged via inflation fluid lumen **18a** into an inflation fluid reservoir (not shown) in order to prevent substantial pressure increase therein.

The distal end of inner shaft **17** may then be vibrated about its longitudinal axis by applying an oscillatory pressure source for periodically changing the pressure of the inflation fluid in balloon **5ab.** Such periodical pressure changes cause corresponding lengthening and shortening of the lengths of balloon **5ab** and inner shaft **17,** thereby traumatizing and/or rupturing the occlusion and thereby opening a passage therethrough.

In the case of a catheter deploying a pre-charged balloon (e.g. the balloon depicted in Fig. 1), the procedure for using the catheter in a crossing procedure is essentially the same as described hereinabove, except for the fact that the inner shaft need not be withdrawn proximally prior to causing the distal-proximal oscillation of the balloon.

Outer shaft **18** may be manufactured by an extrusion and laser cutting process from a polymer, composite or metallic material, such as stainless 316, Nitinol, or nylon, its longitudinal length is generally in the range of 100 to 2000 mm, preferably about 1200 mm, and its diameter is generally in the range of 1 to 2 mm, preferably about 1.2 mm. Inner shaft **17** may be manufactured by an extrusion and laser cutting process from a flexible polymer, composite materials or metallic material, such as pebax, nylon, stainless steel or nitinol, its longitudinal length is generally in the range of 100 to 2000 mm, preferably about 1200 mm, and its diameter is generally in the range of 0.3 to 1 mm, preferably about 0.8 mm. Elastic portions **15** may be obtained by combining one of the above mentioned materials, preferably elastomers, in such portions. A particularly preferred material comprises a blend of nylon and Pebax, for example Pebax 5333, Pebax 6333 etc.

The distal tip **1** of inner shaft **17** may be stiffened by combining stiffening materials such as composite or metals materials therein, and it is preferably has a sharp end for improved penetration. Additionally or alternatively, distal tip **1** may be stiffened by making it thicker relative to other portions of inner shaft **17.**

Fig. 2 schematically illustrates a rapid exchange implementation of the balloon catheter of the invention. The vibration mechanism in this rapid exchange balloon catheter implementation is substantially similar to the mechanism described above with reference to Fig. 1. The catheter's structure mainly differs in that the lumen of its inner shaft may be accessed via a lateral port **23** provided between the proximal and distal ends of the catheter. Inflation fluid lumen **18a** in outer shaft **18** may be filled with pressurized inflation fluids via proximal tube **25** attached thereto. Strain relief portion(s) **22** may be provided over the outer surface of outer shaft **18** for providing additional transitional support and reducing the potential collapse of the catheter's tubes/shafts.

The longitudinal length of inner shaft **17** is generally in the range of 100 to 300 mm, preferably about 120 mm. Proximal tube **25** is made from a flexible polymer, composite or metallic material, such as pebax, nylon, stainless steel or nitinol, having a longitudinal length generally in the range of 100 to 1700 mm, preferably about 1000 mm, and it may be attached to outer shaft **18** by strain relief portion(s) **22** that can be structured by means of over extruded section or heat shrink tube section.

Fig. 3 demonstrates various elastic inner shaft portion implementations that may be used in the balloon catheter of the invention. Elastic portion 15 may be constructed by combining a braided coil section **15a** with an intermediate section thereof. Braided coil, such as manufactured by coil winding processes, may be manufactured from a composite material or as an inner coil with over extrusion of Polymers/elastomers type of materials. The length of the braided coil **15a** combined in inner shaft **17** is generally in range of 3 to 15 mm, preferably about 10 mm.

In an alternative implementation elastic portion **15b** is obtained by embedding coil **33** in an intermediate section thereof. Coil **33** may be embedded in the wall of a portion of inner shaft **17,** or on its outer or inner surface. Coil **33,** such as manufactured by coil winding techniques, may be manufactured from a metallic material and it may be adhered to inner shaft **17** using an acrylic type of adhesive, or embedded in its wall via an over extrusion process. The length of coil **33** is generally in range of 3 to 15 mm, preferably about 10 mm.

Additionally or alternatively, elastic portions **15c** made from one or more elastic material, such as elastomers, polymers, or composite materials, may be embedded in intermediate sections of inner shaft **17.** Elastic portions **155c** may be adhered in intermediate portions of inner shaft **17** using an acrylic, epoxy, or vulcanized type of adhesive, or attached therebetween using an ultrasonic/thermal bonding welding process. The length of elastic portion **15c** is generally in range of 3 to 15 mm, preferably about 10 mm.

Fig. 4 schematically illustrates various distal tip **1** implementations that may be used in the balloon catheter of the invention. Distal tip **1** may be shaped in various forms for achieving a desired rupture effect. A connector (not shown) may be provided at the distal end of the catheter for allowing the physician to choose a suitable tip **1** and connect it thereto. The tip may have a sharp shape as demonstrated in tip **la,** a blunt shape as demonstrated in tips **1b** and **1c,** or a drill like shape as in tips **1d** and **1e.** Tip **1,** such as manufactured by machining, may be manufactured from a metal or composite type of material and its length is generally in range of 1 to 5 mm, preferably about 2 mm.

Figs. 5A and 5B schematically illustrate alternative balloon configurations that may be used in the balloon catheter of the invention. Fig. 5A demonstrates a non-charged implementation of the balloon catheter of the invention wherein both proximal and distal ends, **2a** and **3a,** of balloon **5aa** have conical shapes. The shape of balloon **5aa** is obtained by using a balloon having tapering ends the inner surfaces of which are attached to the outer surfaces of end portions of outer shaft **18** and inner shaft **17.** In the pre-charged example shown in Fig. 5b both proximal and distal portions, **2b** and **3b,** of balloon **5bb** have a rounded, intussuscepted shape which is obtained by attaching the outer surface of the end portions of balloon **5bb** to the outer surface of end portions of outer shaft **18** and of inner shaft **17.** Typically, in order to attach the outer surfaces of the end portions of balloon **5bb** in this way, its distal end **3b** is folded proximally inwardly and its proximal end **2b** is folded distally inwardly.

Balloon 5 may be a non-compliant or semi-compliant or low-compliant balloon, such as manufactured by Interface Assoc, may be manufactured by conventional methods known in the balloon catheter industry from a biocompatible polymer material, preferably from nylon 12 or PET (polyethylene terephthalate). The angle of conical ends of balloon **5,** such as in balloons **5ab** and **5aa,** is generally in range of 10° to 90 °, preferably about 40°.

Fig. 6 schematically illustrates an alternative implementation of the balloon catheter of the invention wherein an auxiliary tube **50,** laterally attached to the outer surface of an end section of outer shaft **18,** is used as a guide wire lumen instead Balloon **5** may be a non-compliant or semi-compliant or low-compliant balloon, such as manufactured by Interface Assoc, may be manufactured by conventional methods known in the balloon catheter industry from a biocompatible polymer material, preferably from nylon 12 or PET (polyethylene terephthalate). The angle of conical ends of balloon **5,** such as in balloons **5ab** and **5aa,** is generally in range of 10° to 90 °, preferably about 40°.

Fig. 6 schematically illustrates an alternative implementation of the balloon catheter of the invention wherein an auxiliary tube **50,** laterally attached to the outer surface of an end section of outer shaft **18,** is used as a guide wire lumen instead of inner shaft **(17).** Auxiliary tube **50** has proximal and distal openings for passing a guide wire therethrough. In this way the balloon catheter of the invention may be manufactured with a single lumen employing the hollow interior of shaft **18** as an inflation fluid lumen. The distal end section of the catheter comprising balloon **5** comprises an inner shaft **67** which proximal end is attached at one or more attachment points **62** located between the proximal ends of balloon **5** and of the catheter to the inner wall of outer shaft **18.** Inner shaft **67** comprises one or more elastic portions **15,** a radiopaque marker **11,** and a tip **1g** adapted for rupturing an occlusion. Auxiliary tube **50** may be manufactured from a flexible polymer or metal and it may be adhered to the outer surface of outer shaft **18** using adhesives or ultrasonic welding/thermal bonding, and its length is generally in range of 100 to 300 mm, preferably about 120 mm.

Figs. 7A to 7F demonstrate one possible procedure for opening a path through an occluded vessel **20** using the balloon catheter of the invention **10.** In this example a non-charged balloon **5aa** is used which has proximal and distal tapering ends attached to the outer surface of a distal portion of outer shaft **18** and of inner shaft **17,** at attachment points **7** and **6,** respectively. Catheter **10** may be advanced towards the treatment site over guide wire **13** threaded through the lumen of inner shaft **17.** Catheter **10** should be placed as near as possible to occlusion **70,** preferably such that distal tip **1** contacts said occlusion. Once catheter **10** is placed in the' treatment site balloon **5aa** may be inflated to a first, anchoring diameter by introducing pressurized inflation fluids (designated by arrows **8a)** via inflation fluid port **11.** Inflation fluids pass via inflation fluid lumen defined between inner wall of outer shaft **18** and the outer surface of inner shaft **17.** In its inflated state (Fig. 7B) lateral sides of balloon **5aa** are pressed against the inner wall **21** of vessel **20,** thereby anchoring it thereto.

After anchoring the balloon in the treatment site the operator manipulates the inflated balloon by releasing a grasping element **14,** thus allowing inner shaft **17** to be moved proximally relative to outer shaft **18.** Inner shaft **17** is retracted proximally and locked into its new location by re-applying grasping element **14** (Fig. 7C). Graduated scale **19,** provided on a proximal portion of inner shaft **17,** may be used to assist the operator in determining the length of inner shaft **17** which has been retracted. Proximal retraction of inner shaft folds the distal end of balloon **5aa** proximally inwardly and shortens the balloon's length and consequently reduces its inflated volume as portions of inflation fluid are discharged therefrom (designated by arrows **8b).**

The discharged portions of inflation fluid may be received by an inflation fluid reservoir (not shown) via inflation fluid port **11** or via a dedicated discharge outlet (not shown).

Alternatively or additionally, the pressure changes in the device may be absorbed utilizing mechanical or pneumatic means (not shown). For example, a gas (e.g., air) bubble (e.g., balloon filled with air) may be placed in outer shaft **18,** which will absorb volumetric changes and thus prevent substantial pressure changes in the shaft **18.** As another example, volumetric changes in shaft 18 may be absorbed by using a movable piston mechanism which can restore a non-pressed state via a spring attached thereto.

The operator may advance the balloon catheter device distally in order to diminish the distance between distal tip **1** and occlusion **70,** if required. This is preferably carried out by partially deflating balloon **5aa,** thereby releasing its anchor in vessel **20,** and advancing the device distally until tip **1** contacts occlusion **70.**

Fig. 7D demonstrates operation of the balloon catheter **10** in a vibrating mode by applying an oscillating pressure source **42** via inflation fluid port **11,** which generates periodical pressure changes in balloon **5aa.** These periodical pressure changes result in periodical lengthening and shortening of balloon **5aa** and elastic portion **15** of inner shaft **17.** The vibrating movement of distal tip **1,** and/or the shockwaves **45** established thereby, fracture occlusion **70** and open a pathway therethrough.

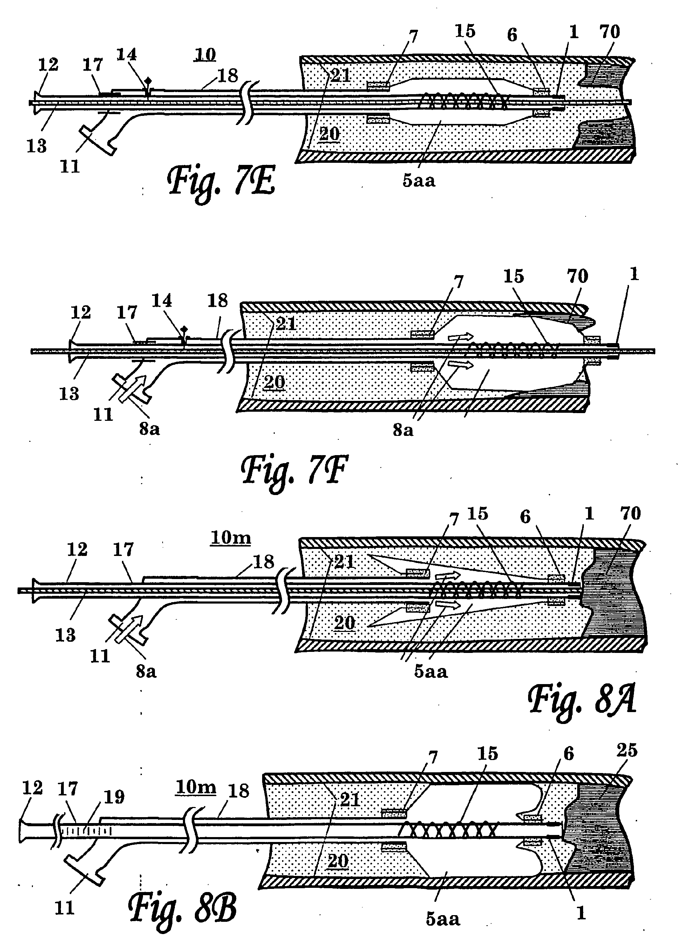

As shown in Fig. 7E guide wire **13** may be then advanced into the fractured occlusion and thereafter the balloon catheter may be also advanced thereinto after deflating balloon **5aa.** At this state the fractured occlusion may be dilatated by inflating balloon 5aa as shown in Fig. 7F.

In the case of a pre-charged balloon catheter, the procedure is essentially the same as described above, except that the step of withdrawing the inner shaft proximally (in order to create an intussusception at the distal end of the balloon) as shown in Fig. 7B, is omitted.

The pressure in balloon **5aa** in its inflated state is generally in the range of 0,2 to 1 MPa (2 to 10 atmospheres), preferably about 0,4 MPa (4 atmospheres), and in its folded state in the range of 0,2 to 1 MPa (2 to 10 atmospheres), preferably about 0,5 MPa (5 atmospheres). Oscillatory pressure source **42** may be implemented in various ways, for example, by utilizing a peristaltic or diaphragm pump, and the pressure oscillations may be controlled by utilizing a solenoid or a revolving eccenter, for instance.

The pressure of the inflation fluid in balloon **5** may be measured by a pressure gauge (not shown) installed at a suitable location along the inflation path, such as in the inflation fluid lumen, for example. Alternatively, the inflation fluid pressure may be obtained utilizing an expansion based indicator (e.g., a flexible part which reacts to pressure by elongating) or by mechanical displacement indicator (e.g., indicator which records the longitudinal movement of the cylinder and translates it to pressure changes).

In one embodiment, balloon **5** may be attached to the catheter in such a way that said balloon is twisted along its longitudinal length. Such a longitudinal twist may be obtained by slightly rotating attaching one of the balloon's ends and attaching it to its respective attachment point. In this way the inflation of balloon **5** will apply a rotational force on the inner shaft **17** attached thereto which cause elastic portions **15** thereof to twist and thus provide a drilling effect by slightly rotating tip **1** about its axis. It should be noted that a similar effect is also obtained when using spring like elements to implement elastic portions **15** due to the twist induced by such elements during stretch and compression thereof.

Another example for a procedure for opening a path through an occluded vessel **20** that may be performed with a modified balloon catheter **10m** of the invention will be now described with reference to Figs. 8A and 8B. In this example inner shaft **17** is affixed to outer shaft **18** (e.g., using a suitable adhesive), and balloon **5aa** is folded proximally (backwardly) thus forming an arrow-like shape which tapers towards its distal attachment point **6,** as shown in Fig. 8A. This folded state may be retained by folding the balloon into this folded state under heat and/or pressure (e.g., while folding the balloon in the manufacturing process the balloon will maintain its shape if the "wings" of the folded-jacket will remain tight).

Catheter **10m** may be advanced towards the treatment site over guide wire **13** threaded through the lumen of inner shaft **17.** Catheter **10m** is placed adjacent to occlusion **70,** preferably such that distal tip **1** contacts said occlusion. Once catheter **10m** is placed in the treatment site balloon **5aa** may be inflated by introducing pressurized inflation fluids (designated by arrows **8a)** via inflation fluid port **11.** Inflation fluids pass via inflation fluid lumen defined between inner wall of outer shaft 18 and the outer surface of inner shaft **17.** In its inflated state (Fig. 7B) lateral sides of the backwardly folded balloon **5aa** are pressed against the inner wall **21** of vessel **20,** thereby anchoring it thereto. Due to its initial folded state the distal end of the inflated balloon gains a rounded, intussuscepted shape, as shown in Fig. 8B.

After anchoring the balloon in the treatment site the physician may operate the device in a vibrating' mode by applying an oscillating pressure source **42** via inflation fluid port **11,** open a passage through the occlusion and perform balloon dilatation in needed, as was previously described with reference to Figs. 7D to 7F.

The pressure in balloon **5aa** in its inflated state is generally in the range of 0,2 to 1 MPa (2 to 10 atmospheres), preferably about 0,4 MPa (4 atmospheres), and in its folded state in the range of 0,2 to 1 MPa (2 to 10 atmospheres), preferably about 0,5 MPa (5 atmospheres).

While in the figures an inner shaft **17** comprising an elastic portion is shown, it should be understood that the entire inner shaft may be manufactured from an elastic material.

It should be noted that balloon 5 may be operated also manually or mechanically in procedures such as described hereinabove. For example, the operator can carry out the occlusions opening steps (or portion thereof) of the procedure by pulling inner shaft **17** proximally and releasing. Such operation will cause proximal and distal movements of tip **1** and assist in rupturing occlusion **70.** Similarly, mechanical means (not shown e.g., mechanical actuator which can be used on the proximal end of the catheter to reciprocatingly withdraw the inner shaft and release against the flexibility of the balloon accumulating pressure change) may be used to introduce such movements of tip **1.**

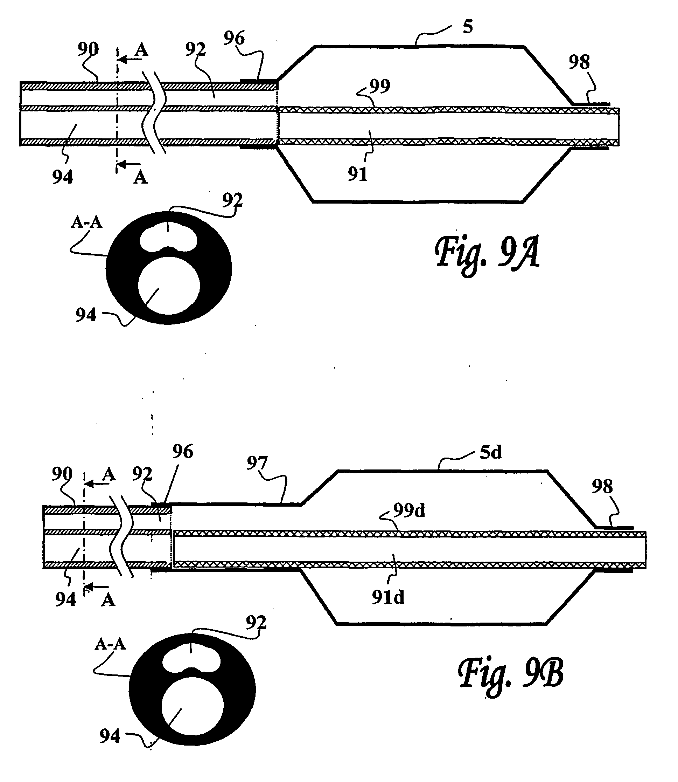

Fig. 9A illustrates one embodiment of the invention utilizing bi-lumen catheter tubing along at least a portion of the overall catheter length. In this figure, the proximal end of balloon **5** is attached to the external surface of bi-lumen conduit **90** at proximal attachment point **96,** said bi-lumen conduit comprising two parallel lumens: inflation fluid lumen **92** and guidewire lumen **94.** A cross-sectional view of the bi-lumen conduit taken at level A-A that shows the relative arrangement of the two lumens is provided in -the lower part of this figure. While inflation fluid lumen **92** ends at the proximal balloon attachment point **96,** guidewire lumen **94** continues beyond the proximal attachment point **96** of balloon **5,** said lumen becoming continuous with the guidewire lumen **91** of distal conduit **99.** The outer surface of said distal conduit, which contains an elastically deformable region, provides a distal balloon attachment point **98.**

In the embodiment of the catheter shown in Fig. 9B, the modified balloon, **5d,** has an elongated proximal neck, **97.** The increase in length of the balloon in this embodiment permits the use of a longer distal conduit **99d.** All the other elements in this embodiment are the same as those shown in Fig. 9A.

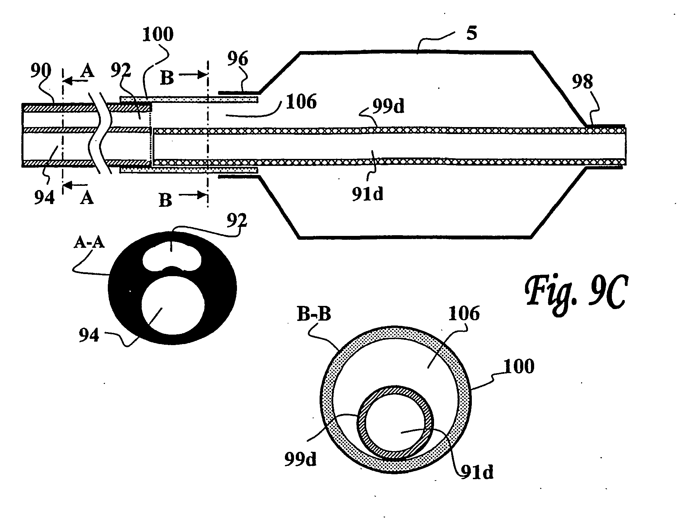

Fig. 9C illustrates another embodiment of the bi-lumen configuration described hereinabove. In this case, the catheter further, comprises a connecting tube segment **100** positioned between the conduit proximal portion (i.e. bi-lumen conduit) **90** and the proximal attachment point of the balloon **96.** Said connecting tube segment, shown in cross-sectional view in the lower right portion of Fig. 9C, contains two concentrically arranged conduits: an outer conduit having a lumen **106** that is in fluid communication with the fluid passage lumen **92** of the proximal bi-lumen conduit **90,** and an inner conduit formed by the elastic section-containing distal conduit **99d,** the lumen **91d** of which is in fluid communication with the guidewire lumen **94** of said bi-lumen conduit **90.** As seen in the figure, the presence of connecting tube segment **100** permits the use of a longer distal conduit **99d** than is possible in the embodiment depicted in Fig. 9A.

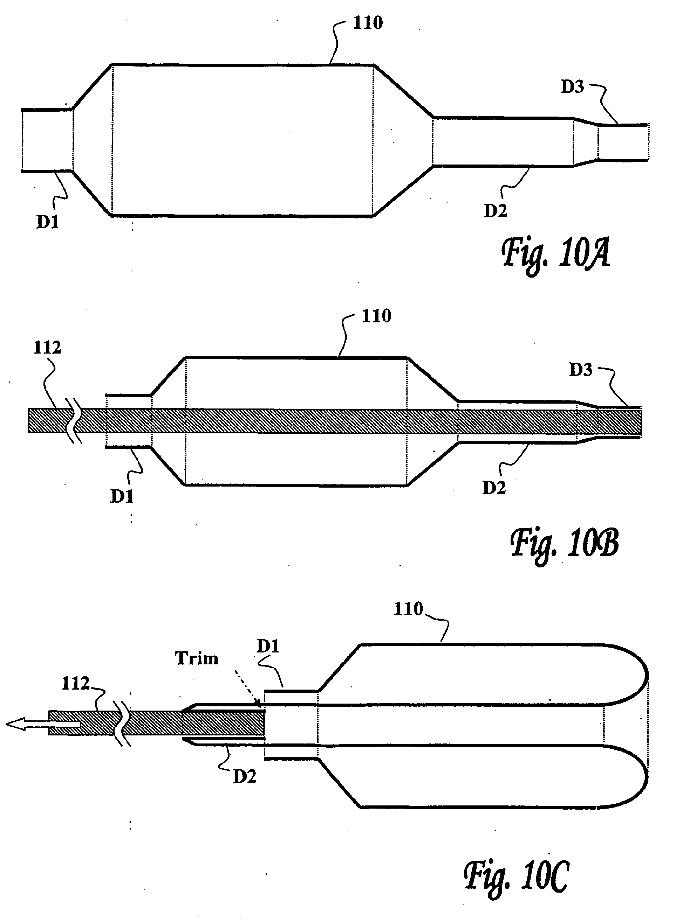

As mentioned hereinabove, there exist several different procedures for attaching the balloon to the catheter shafts that may be employed in the manufacture of the devices of the present invention. One example of such a procedure, which is illustrated in Figs. 10A to 10E, is known as 'flipped distal neck bonding'. As shown in Fig. 10A, the balloon **110** is blown from a length of standard tubing material (e.g. 0.6 mm diameter nylon 12 and/or a Pebax material).

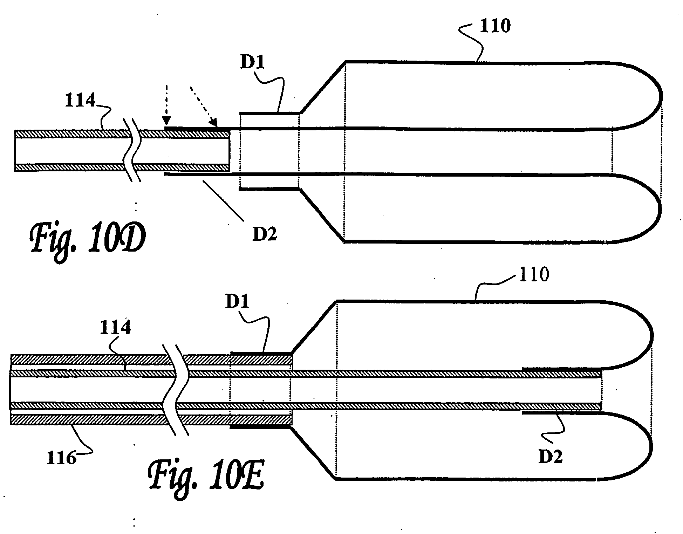

Following balloon blowing, the tubing that is continuous with the proximal and distal extremities of the balloon forms three distinct areas, each having different diameters. Thus, on the proximal side of the balloon, the' tubing has inner diameter **D1,** said diameter matching the outer catheter shaft that is to be connected thereto. The region immediately distal to the balloon has diameter **D2,** said diameter matching the outer diameter of the inner catheter shaft. Finally, the distal-most region has a diameter **D3** that is smaller than **D2.** The purpose of this undersized region, as shown in Fig. 10B, is to permit bonding to a mandrel **112** that is inserted through the lumen of balloon **110.** The next stage, as shown in Figure 10C is the pulling of mandrel **112** in a proximal direction (as shown by the arrow). Since the mandrel is firmly bonded to the distal-most portion of the balloon, the pulling motion results in inversion and intussusception of the distal portion of the balloon through its lumen. The mandrel is then trimmed at the point indicated by the arrow and removed. The next stage, as shown in Fig. 10D, is the insertion of the inner tube **114** into the portion of the tubing having diameter **D2** that was originally located (e.g. in Fig. 10A) distal to the expanded portion of the balloon **110.** The inner tube is bonded to inner tube **114** along the section of the tubing marked with arrows. Fig. 10E shows balloon **110** following the final stages of the procedure, wherein the balloon has been rolled back into its original position, and the outer tube **116** has been bonded into the distal neck of the balloon' (the region having diameter **D1).** It may be seen from this figure that the balloon produced by this technique is pre-charged, having a distal intussusception.

All of the abovementioned parameters are given by way of example only, and may be changed in accordance with the differing requirements of the various embodiments of the present invention. Thus, the abovementioned parameters should not be construed as limiting the scope of the present invention in any way. In addition, it is to be appreciated that the different shafts and tubes, and other members, described hereinabove may be constructed in different shapes (e.g. having oval, square etc. form in plan view) and sizes from those exemplified in the preceding description.

The above examples and description have of course been provided only for the purpose of illustration, and are not intended to limit the invention in any way. As will be appreciated by the skilled person, the invention can be carried out in a great variety of ways, employing more than one technique from those described above, all without exceeding the scope of the invention.

## Claims

1. A balloon catheter comprising:
a hollow inner shaft (17) disposed within a hollow outer shaft (18) such that the distal end of the inner shaft extends beyond the distal end of the outer shaft, wherein the lumen of said inner shaft is suitable for allowing the passage of a guidewire through all or part of its length;
a balloon (5ab) attached at its proximal end to said outer shaft and at its distal end to said inner shaft; and
means for the introduction of an inflation fluid into the annular space formed between the inner surface of the outer shaft and the outer surface of the inner shaft and therefrom into the lumen of said balloon, and for the removal thereof;
**characterised in that** the inner shaft is constructed such that it comprises at least one portion (15) that is elastically deformable such that following radial expansion of the balloon to a first expanded state,
said inner shaft is capable of responding to further longitudinal expansion of the balloon to a second expanded state by increasing its length from a resting value, and of responding to subsequent partial deflation back to said first expanded state by reducing its length back to said resting value.

2. The balloon catheter according to claim 1, wherein, following radial expansion of the balloon to a first expanded state, said elastically deformable portion of the inner shaft is capable of responding to further longitudinal expansion of the balloon to a second expanded state by increasing its length from a resting value, and of responding to subsequent partial deflation back to said first expanded state by reducing its length back to said resting value.

3. The balloon catheter according to claim 1, wherein the distance between the points of attachment of the balloon to the inner and outer shafts is less than the overall length of the balloon.

4. The balloon catheter according to claim 1, wherein the at least one elastically deformable portion is constructed of a blend of nylon and Pebax.

5. The balloon catheter according to claim 1, wherein the balloon is attached at its distal end in an inverted manner, such that the external surface of said balloon is in contact with, and attached to, the outer surface of the inner shaft.

6. The balloon catheter according to claim 1, wherein the balloon is attached at its distal end in a non-inverted manner, such that the internal surface of said balloon is in contact with, and attached to, the outer surface of the inner shaft.

7. The -balloon catheter according to claim 1, wherein said catheter is constructed as an over-the-wire catheter.

8. The balloon catheter according to claim 1, wherein the inner shaft is capable of being moved along its longitudinal axis in relation to the outer shaft, and wherein said catheter further comprises means for immobilizing said inner shaft.

9. The balloon catheter according to claim 1, wherein said catheter is constructed as a rapid exchange catheter.

## Patentansprüche

1. Ballonkatheter, der Folgendes umfasst:
einen hohlen Innenschaft (17), der in einem hohlen Außenschaft (18) angeordnet ist, so dass sich das distale Ende des Innenschafts über das distale Ende des Außenschafts hinaus erstreckt, wobei das Lumen des Innenschafts geeignet ist, den Durchtritt eines Führungsdrahts durch seine gesamte Länge oder einen Teil seiner Länge zuzulassen;
einen Ballon (5ab), der an seinem proximalen Ende an dem Außenschaft und an seinem distalen Ende an dem Innenschaft angebracht ist; und
Mittel zum Einbringen einer Inflationsflüssigkeit in den ringförmigen Raum, der zwischen der Innenfläche des Außenschafts und der Außenfläche des Innenschafts gebildet wird, und von dort in das Lumen des Ballons und zum Abziehen dieser Flüssigkeit;
**dadurch gekennzeichnet, dass** der Innenschaft derart konstruiert ist, dass er mindestens einen Teil (15) umfasst, der elastisch verformbar ist, so dass nach einer radialen Ausdehnung des Ballons in einen ersten ausgedehnten Zustand der Innenschaft auf eine weitere Längsausdehnung des Ballons in einen zweiten ausgedehnten Zustand reagieren kann, indem er seine Länge von einem Ruhewert vergrößert, und auf eine anschließende Teildeflation zurück in den ersten ausgedehnten Zustand reagieren kann, indem er seine Länge zurück in den Ruhewert verkleinert.

2. Ballonkatheter nach Anspruch 1, wobei nach einer radialen Ausdehnung des Ballons in einen ersten ausgedehnten Zustand der elastisch verformbare Teil des Innenschafts auf eine weitere Längsausdehnung des Ballons in einen zweiten ausgedehnten Zustand reagieren kann, indem er seine Länge von einem Ruhewert vergrößert, und auf eine anschließende Teildeflation zurück in den ersten ausgedehnten Zustand reagieren kann, indem er seine Länge zurück in den Ruhewert verkleinert.

3. Ballonkatheter nach Anspruch 1, wobei der Abstand zwischen den Punkten der Anbringung des Ballons an dem Innenschaft und dem Außenschaft geringer als die Gesamtlänge des Ballons ist.

4. Ballonkatheter nach Anspruch 1, wobei der mindestens eine elastisch verformbare Teil aus einem Gemisch von Nylon und Pebax konstruiert ist.

5. Ballonkatheter nach Anspruch 1, wobei der Ballon an seinem distalen Ende invertiert angebracht ist, so dass die Außenfläche des Ballons mit der Außenfläche des Innenschafts in Kontakt steht und an dieser angebracht ist.

6. Ballonkatheter nach Anspruch 1, wobei der Ballon an seinem distalen Ende nicht-invertiert angebracht ist, so dass die Innenfläche des Ballons mit der Außenfläche des Innenschafts in Kontakt steht und an dieser angebracht ist.

7. Ballonkatheter nach Anspruch 1, wobei der Katheter als ein Over-the-Wire-Katheter konstruiert ist.

8. Ballonkatheter nach Anspruch 1, wobei der Innenschaft entlang seiner Längsachse in Bezug auf den Außenschaft bewegt werden kann und wobei der Katheter weiterhin Mittel zum Immobilisieren des Innenschafts umfasst.

9. Ballonkatheter nach Anspruch 1, wobei der Katheter als ein Rapid-Exchange-Katheter konstruiert ist.

## Revendications

1. Cathéter à ballonnet comprenant :
une tige interne creuse (17) disposée à l'intérieur d'une tige externe creuse (18) de telle sorte que l'extrémité distale de la tige interne s'étend au-delà de l'extrémité distale de la tige externe, la lumière de ladite tige interne étant appropriée pour autoriser le passage d'un fil-guide sur la totalité ou une partie de sa longueur ;
un ballonnet (5ab) fixé par son extrémité proximale à ladite tige externe et par son extrémité distale à ladite tige interne ; et
un moyen permettant l'introduction d'un liquide de gonflage dans l'espace annulaire formé entre la surface intérieure de la tige externe et la surface extérieure de la tige interne et, par conséquent, dans la lumière dudit ballonnet, et permettant le retrait dudit liquide de gonflage ;
**caractérisé en ce que** la tige interne est conçue de telle manière qu'elle comprend au moins une partie (15) élastiquement déformable, conçue de façon qu'à la suite d'une extension radiale du ballonnet vers un premier état étendu, ladite tige interne est capable de réagir à une autre extension longitudinale du ballonnet vers un second état étendu en augmentant sa longueur depuis une valeur de repos, et de réagir à un dégonflement partiel ultérieur vers ledit premier état étendu en ramenant sa longueur à ladite valeur de repos.

2. Cathéter à ballonnet selon la revendication 1, dans lequel, à la suite d'une extension radiale du ballonnet vers un premier état étendu, ladite partie élastiquement déformable de la tige interne est capable de réagir à une autre extension longitudinale du ballonnet vers un second état étendu en augmentant sa longueur depuis une valeur de repos, et de réagir à un dégonflement partiel ultérieur vers ledit premier état étendu en ramenant sa longueur à ladite valeur de repos.

3. Cathéter à ballonnet selon la revendication 1, dans lequel la distance entre les points de fixation du ballonnet aux tiges interne et externe est inférieure à la longueur globale du ballonnet.

4. Cathéter à ballonnet selon la revendication 1, dans lequel la ou les parties élastiquement déformables sont constituées d'un mélange de nylon et de Pebax.

5. Cathéter à ballonnet selon la revendication 1, dans lequel le ballonnet est fixé à son extrémité distale de manière inversée, de telle sorte que la surface extérieure dudit ballonnet est en contact avec et est fixée à la surface extérieure de la tige interne.

6. Cathéter à ballonnet selon la revendication 1, dans lequel le ballonnet est fixé à son extrémité distale d'une manière non inversée, de telle sorte que la surface intérieure dudit ballonnet est en contact avec, et est fixée à la surface extérieure de la tige interne.

7. Cathéter à ballonnet selon la revendication 1, dans lequel ledit cathéter est conçu comme un cathéter sur fil-guide.

8. Cathéter à ballonnet selon la revendication 1, dans lequel la tige interne peut être déplacée le long de son axe longitudinal par rapport à la tige externe, et dans lequel ledit cathéter comprend en outre un moyen permettant d'immobiliser ladite tige interne.

9. Cathéter à ballonnet selon la revendication 1, dans lequel ledit cathéter est conçu comme un cathéter à échange rapide.
